# DEMANDE DE BREVET EUROPEEN

(11) **EP 0 694 303 A1**
(43) Date de publication de la demande: **31.01.1996**
(21) Numéro de dépôt: 95401737.2
(22) Date de dépôt: 24.07.1995
(51) Int. Cl.: A61K 31/445

(54) **Utilisation de l'eliprodil et de ses énantiomères pour la préparation de médicaments utiles dans la prévention des neuropathies induites par des agents anticancéreux, e.g. le paclitaxel**

(30) Priorité: 29.07.1994 FR 9409409
(71) Demandeur: SYNTHELABO, F-92350 Le Plessis Robinson (FR)
(72) Inventeur: Lekieffre, Delphine, F-75014 Paris (FR); Benavides, Jésus, F-92290 Chatenay Malabry (FR); Scatton, Bernard, F-91120 Villebon sur Yvette (FR); George, Pascal, F-78730 Saint Arnoult en Yvelines (FR)
(74) Mandataire: Thouret-Lemaitre, Elisabeth

(57) **Abrégé**

Utilisation de l'éliprodil et de ses énantiomères pour la préparation de médicaments utiles dans la prévention des neuropathies périphériques induites par des agents anticancéreux et compositions pharmaceutiques, constituées d'éliprodil ou de l'un de ses énantiomères et d'un agent anticancéreux, pour des traitements anticancéreux sans risques d'atteintes du système nerveux périphérique.

## Description

La présente invention a pour objet l'utilisation de l'éliprodil et de ses énantiomères pour la préparation de médicaments utiles dans la prévention des neuropathies périphériques dues à des chimiothérapies anticancéreuses, ainsi que des compositions pharmaceutiques à base d'éliprodil ou de l'un de ses énantiomères et d'agents anticancéreux, et leur application comme médicaments anticancéreux évitant les risques de neuropathies périphériques.
L'éliprodil et ses énantiomères peuvent être utlisés sous forme de base ou de sel d'addition à un acide pharmaceutiquement acceptable.

L'éliprodil, de formule (I) 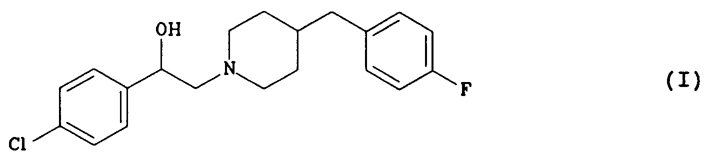
est un composé connu, dont les propriétés neuroprotectrices sont décrites dans le brevet européen n° 0 109 317.

Ses énantiomères sont décrits dans le certificat d'utilité FR 89 04835.

De nouvelles recherches ont mis en évidence l'activité neurotrophe de l'éliprodil. Ces recherches ont été poursuivies afin d'évaluer l'intérêt de ce composé dans la prévention des neuropathies périphériques induites par des agents anticancéreux.
Certains agents anticancéreux sont en effet connus pour induire, aux doses thérapeutiques, des altérations pathologiques des fibres nerveuses périphériques sensorielles et motrices, ce qui limite leur utilisation.

A cet égard, le paclitaxel (TAXOL®), composé actif dans le traitement des mélanomes malins et des carcinomes ovariens, est particulièrement représentatif, car il induit des neuropathies sensorielles qui affectent principalement les fibres de petit diamètre (L.B. LIPTON et col., Neurology, vol. 39, 368-373, 1989).
Le paclitaxel est un dérivé alcaloïde qui se lie à la tubuline, entraînant ainsi l'agrégation des microtubules. Cette perturbation est visible au niveau des axones et des cellules de Schwann et conduit à une dégénérescence axonale et à une démyélinisation.

Une neuropathie au paclitaxel, reproduisant la pathologie humaine, peut aisément être induite chez l'animal.
Ce modèle animal permet donc d'évaluer l'efficacité de diverses stratégies pharmacologiques vis-à-vis des neuropathies périphériques dues à des chimiothérapies.

L'évaluation des effets préventifs de l'éliprodil vis-à-vis des neuropathies induites par le paclitaxel a été réalisée in vivo selon le protocole suivant :

Le paclitaxel a été administré à la dose de 20 mg/kg par voie intrapéritonéale, pendant 6 jours consécutifs, à des souris OF1 (Iffa Credo), pesant 10 à 12 g. Il a été injecté sous forme de suspension dans une solution à 10 % de Crémophor® EL dans du sérum physiologique, sous un volume de 0,1 ml/10 g de poids corporel.

Le chlorhydrate d'éliprodil a été préparé sous forme de suspension dans une solution aqueuse à 0,1 % de Tween-80 et administré par voie intrapéritonéale à la dose de 1 mg/kg, 2 fois par jour pendant 8 jours, la première injection ayant lieu 24 heures avant celle du paclitaxel.

Les animaux ont été répartis en 4 groupes expérimentaux :
- groupe des témoins absolus, ne recevant ni paclitaxel ni éliprodil ;
- groupe des témoins traités, recevant uniquement de l'éliprodil ;
- groupe traité uniquement par le paclitaxel ;
- groupe traité par le paclitaxel et l'éliprodil.

5 jours après la dernière administration de paclitaxel, les animaux ont été soumis à un test de sensibilité à la douleur (test de "tail-flick"). Ce test consiste à placer l'extrémité de la queue (2 mm) de la souris entre un faisceau lumineux (100 watts) et une cellule photosensible, l'ensemble étant couplé à un compteur automatique. On note le temps au bout duquel l'animal retire sa queue du faisceau lumineux (temps de latence), l'augmentation du temps de latence étant un bon indice de l'atteinte sensorielle, comme l'ont décrit S.C. Apfel, R.B. Lipton, J.C. Arezzo, J.A. Kessler, dans Ann. Neurol., (1991), 29, n°1, 87-90.

Les résultats suivants ont été obtenus :
- les souris témoins présentent un temps de latence de 3 secondes qui n'a pas été modifié par le traitement à l'éliprodil.
- chez les souris traitées au paclitaxel, le temps de latence est augmenté de 45 %.
- chez les souris recevant le traitement paclitaxel + éliprodil, le temps de latence est identique à celui des groupes témoins.

Ces résultats montrent que le traitement associant l'éliprodil au paclitaxel s'oppose totalement à la perturbation sensorielle induite par le paclitaxel.

Il apparaît que l'éliprodil prévient les troubles sensoriels liés à l'administration du paclitaxel.
L'éliprodil et ses énantiomères peuvent donc être utilisés pour la préparation de médicaments utiles dans la prévention des neuropathies induites par le paclitaxel ou tout autre agent anticancéreux inducteur de neuropathies, par exemple le cisplatine, la vinblastine, la vincristine ou la vindésine.

Il peut être présenté, en association avec des excipients appropriés, sous toute forme galénique convenant à une administration orale, parentérale ou locale, par exemple sous forme de comprimés, de gélules, de solutions ou de patch transdermique, contenant 0,1 à 100 mg d'éliprodil ou de l'un de ses énantiomères par dose unitaire. Il peut être administré préalablement et/ou simultanément au traitement anticancéreux, à raison de 1 à 3 doses unitaires par jour.

Il peut également être administré sous forme de compositions pharmaceutiques constituées d'éliprodil ou de l'un de ses énantiomères et d'un agent anticancéreux, en association avec des excipients appropriés et présentées sous toute forme galénique telle qu'indiquée ci-dessus. Ces compositions permettent le traitement des cancers tout en évitant les atteintes du système nerveux périphérique.
Elles contiennent 0,1 à 100 mg d'éliprodil ou de l'un de ses énantiomères, et sont administrées selon le mode d'administration prescrit pour l'agent anticancéreux.

## Revendications

1. Utilisation de l'éliprodil et de ses énantiomères, sous forme de base ou de sel d'addition à un acide pharmaceutiquement acceptable, pour la préparation d'un médicament utile dans la prévention des neuropathies périphériques induites par des agents anticancéreux.

2. Composition pharmaceutique caractérisée en ce qu'elle est constituée d'un agent anticancéreux et d'éliprodil ou de l'un de ses énantiomères, sous forme de base ou de sel d'addition à un acide pharmaceutiquement acceptable, associés à des excipients appropriés.

3. Composition pharmaceutique selon la revendication 2, caractérisée en ce que l'agent anticancéreux est le paclitaxel.
